# EUROPEAN PATENT APPLICATION

(11) **EP 2 455 470 A1**
(43) Date of publication of application: **23.05.2012**
(21) Application number: 10799947.6
(22) Date of filing: 20.07.2010
(51) Int. Cl.: C12N 15/09, C12Q 1/68

(54) **METHOD FOR AMPLIFYING NUCLEIC ACID**

(30) Priority: 17.07.2009 JP 2009168899
(71) Applicant: Toyo Seikan Kaisha, Ltd., Tokyo 100-8522 (JP)
(72) Inventor: TOYAMA Masafumi, Yokohama-shi Kanagawa 240-0062 (JP)
(74) Representative: Chapman, Paul Gilmour
(86) International application number: PCT/JP2010/062157
(87) International publication number: WO 2011/007889

(57) **Abstract**

Disclosed is a nucleic acid amplification method which is based on a new principle and enables to amplify a nucleic acid having a specific nucleotide sequence in a simple manner, within a short time and with efficiency. The nucleic acid amplification method comprises the steps of: (a) obtaining a linear DNA fragment by performing a DNA polymerase elongation reaction by using a template DNA comprising a base sequence to be amplified and a primer pair comprising a primer having a base sequence complementary to a region adjacent to a 3' end of the base sequence to be amplified and a chemically modified 3' end; and (b) performing a strand displacement-type DNA polymerase elongation reaction on a circular single-stranded DNA comprising the base sequence to be amplified and serving as a template, with a 3' end of the linear DNA. fragment obtained in (a) serving as an origin of replication.

SEQUENCE LISTING

<110>  TOYO SEIKAN KAISHA, Ltd

<120>  Method of Amplifying nucleic acid(s)

<130>  Y1R0359

<160>  14

<170>  PatentIn version 3.3

<210>  1
<211>  60
<212>  DNA
<213>  Artificial

<220>
<223>  designed polynucleotide

<400>  1
agagctatga tacgccactg tcccttttgt aatgtccgct gagaaggacg cacacatgat      60


<210>  2
<211>  20
<212>  DNA
<213>  Artificial

<220>
<223>  desinged polynucleotide

<400>  2
tcatagctct atcatgtgtg                                                  20


<210>  3
<211>  60
<212>  DNA
<213>  Artificial

<220>
<223>  desinged polynucleotide

<400>  3
agagctatga ccaccgacca tctatgactg ggcatgcacc gagaaggacg cacacatgat      60


<210>  4
<211>  20
<212>  DNA
<213>  Artificial

<220>
<223>  desinged polynucleotide

<400>  4
ccaccgacca tctatgactg                                                  20


<210>  5
<211>  20
<212>  DNA
<213>  Artificial

<220>
<223>  designed polynucleotide

<400>  5
ggcatgcacc gagaaggacg                                                  20

<210>  6
<211>  20
<212>  DNA
<213>  Artificial

<220>
<223>  desinged polynucleotide

<400>  6
gtgcgggtgt tgaatgattt                                                    20


<210>  7
<211>  20
<212>  DNA
<213>  Artificial

<220>
<223>  designed polynucleotide

<400>  7
gctaaccaat tcctaggcag                                                    20


<210>  8
<211>  20
<212>  DNA
<213>  Artificial

<220>
<223>  designed polynucleotide


<220>
<221>  misc_feature
<222>  (20)..(20)
<223>  inosine

<400>  8
ccaccgacca tctatgactn                                                    20


<210>  9
<211>  20
<212>  DNA
<213>  Artificial

<220>
<223>  designed polynucleotide


<220>
<221>  misc_feature
<222>  (20)..(20)
<223>  inosine

<400>  9
ggcatgcacc gagaaggacn                                                    20


<210>  10
<211>  60
<212>  DNA
<213>  Artificial

<220>
<223>  designed polynucleotide

<400>  10
agagctatga tacgccactg tcccttacgc cactgtccct gagaaggacg cacacatgat      60


<210>  11
<211>  60
<212>  DNA
<213>  Artificial

<220>
<223>  designed polynucleotide

<400>  11
agagctatga tttgtaatgt ccgcttttgt aatgtccgct gagaaggacg cacacatgat      60


<210>  12
<211>  196
<212>  DNA
<213>  lambda phage

<400>  12
gtgcgggtgt tgaatgattt ccagttgcta ccgattttac atattttttg catgagagaa      60

tttgtaccac ctcccaccga ccatctatga ctgtacgcca ctgtccctag gactgctatg     120

tgccggagcg gacattacaa acgtccttct cggtgcatgc cactgttgcc aatgacctgc     180

ctaggaattg gttagc                                                     196


<210>  13
<211>  65
<212>  DNA
<213>  Artificial

<220>
<223>  designed polynucleotide

<400>  13
agagctatga tacgccactg tcccttttgt aatgtccgct gactggagaa ggacgcacac      60

atgat                                                                  65


<210>  14
<211>  21
<212>  DNA
<213>  Artificial

<220>
<223>  designed polynucleotide

<400>  14
tcatagctct atcatgtgtg g                                                21

## Description

### Technical Field

The present invention relates to a method for amplifying a nucleic acid, especially to a method for amplifying a nucleic acid using a combination of a chemically modified primer and a circular single-stranded DNA, a method for detecting whether or not a target gene is present by using the above method, and a detection kit to be used for the above method.

### Background Art

Currently, in various fields including research institutes, medical facilities, inspection agencies, and others, a large number of analysis and detection methods based on a specific base sequence of a target gene are employed. For example, in the case of analyzing and detecting the presence of a pathogenic microorganism or a minute creature such as a pathogen or allergens including fungus, ticks, and the like; virus; pollen; or the like included in a sample (body fluid or cell fragment) derived from a biological body, such as human and animals, or a sample derived from the environment, methods for detecting a specific base sequence of a target gene included in such detection targets have been used. These methods for detecting nucleic acids (DNA, RNA) can be performed in a shorter period of time and at a higher sensitivity than methods for detecting a protein. Furthermore, even if the amount of nucleic acid originally included in a sample is below a detection limit, the nucleic acid can be amplified relatively easily and specifically using a cell-free system. With these advantages, by using a method for amplifying a nucleic acid or in combination with a method for amplifying a nucleic acid, many methods for detecting a specific base sequence of a target gene have been developed.

One of the nucleic acid amplification techniques which are most commonly used at the present time is a PCR method in which a cycle of template denaturation, primer annealing to the template, and a DNA polymerase elongation reaction is performed several tens of times by use of a temperature cycle so as to amplify a nucleic acid in a region sandwiched by a primer pair. However, the method for amplifying a nucleic acid by use of the temperature cycle has such problems that an instrument for controlling the temperature cycle (thermal cycler or the like) is expensive and that it is required to examine and set an optimal temperature cycle for nucleic acid amplification. Hence, in recent years, a method for amplifying a nucleic acid has been studied in which a DNA polymerase elongation reaction is carried out under a constant temperature condition without using a temperature cycle. A representative example of such a method is a LAMP (Loop-Mediated Isothermal Amplification) method (Patent Document 1). In addition, another method for amplifying a nucleic acid has been developed which uses a combination with a circular single-stranded DNA for overcoming problems associated with costs, design and operation of a detection method, detection sensitivity, and the like (Patent Document 2).

However, the above-described methods are sometimes still not necessarily sufficient in terms of detection sensitivity, when a sample contains only a trace amount of a nucleic acid to be detected. Accordingly, a novel method for amplifying a nucleic acid and a novel method for detecting a nucleic acid are required which are further improved in amplification efficiency and detection sensitivity.

### Citation List

### Patent Literatures

Patent Literature 1: International Patent Application Publication No. W02000/28082
Patent Literature 2: International Patent Application Publication No. W02008/026719

### Summary of Invention

### Technical Problem

An object of the present invention is to provide a method for amplifying a nucleic acid based on a novel principle in which a nucleic acid having a specific base sequence can be efficiently amplified easily and in a short period of time, and a method for detecting a nucleic acid using the method.

### Solution to Problems

The inventor of the present invention has discovered that the above-described problems can be solved by performing a series of DNA polymerase elongation reaction for a template nucleic acid molecule having a specific base sequence with the use of a combination of a primer having a base sequence complementary to a region adjacent to a 3' end of the base sequence and a chemically modified 3' end and a circular single-stranded DNA comprising the specific base sequence. This discovery has led to the completion of the present invention.

Specifically, the present invention provides
a method for amplifying a nucleic acid, comprising:
(a) obtaining a linear DNA fragment by performing a DNA polymerase elongation reaction by using a template DNA comprising a base sequence to be amplified and a primer pair comprising a primer having a base sequence complementary to a region adjacent to a 3' end of the base sequence to be amplified and a chemically modified 3' end; and
(b) performing a strand displacement-type DNA polymerase elongation reaction on a circular single-stranded DNA comprising the base sequence to be amplified and serving as a template, with a 3' end of the linear DNA fragment obtained in (a) serving as an origin of replication.

Moreover, the present invention provides
a nucleic acid amplification kit comprising:
(i) a primer pair comprising a primer having a base sequence complementary to a region adjacent to a 3' end of a base sequence to be amplified and a chemically modified 3' end;
(ii) a circular single-stranded DNA comprising the base sequence to be amplified;
(iii) a strand displacement-type DNA polymerase; and
(iv) dNTP.

Furthermore, the present invention provides
a method for detecting a double-stranded target nucleic acid molecule comprising:
(a) adding a primer pair comprising a primer having a base sequence complementary to a region adjacent to a 3' end of a target base sequence of a target nucleic acid molecule and a chemically modified 3' end; a circular single-stranded DNA comprising the target base sequence; a strand displacement-type DNA polymerase; and dNTP to a sample to be subjected to detection, and performing an enzymatic reaction at a temperature at which the strand displacement-type DNA polymerase retains its activity;
(b) checking whether or not a nucleic acid is amplified in the sample subjected to the enzymatic reaction; and
(c) in the case where a nucleic acid is amplified, determining that a double-stranded target nucleic acid molecule is present in the sample subjected to detection.

In addition, the present invention provides
a method for detecting a single-stranded target nucleic acid molecule comprising:
(a) adding a primer having a base sequence complementary to a region adjacent to a 3' end of a target base sequence of a target nucleic acid molecule and a chemically modified 3' end; a primer having a base sequence of a region adjacent to a 3' end of a target base sequence of a region different from a region of the target base sequence of the target nucleic acid molecule and a chemically modified 3' end; a circular single-stranded DNA comprising the target base sequences; a strand displacement-type DNA polymerase; and dNTP to a sample to be subjected to detection, and performing an enzymatic reaction at a temperature at which the strand displacement-type DNA polymerase retains its activity;
(b) checking whether or not a nucleic acid is amplified in the sample subjected to the enzymatic reaction; and
(d) in the case where a nucleic acid is amplified, determining that a single-stranded target nucleic acid molecule is present in the sample subjected to detection.

Moreover, the present invention provides
a double-stranded target nucleic acid molecule detection kit, comprising:
(i) a primer pair comprising a primer having a base sequence complementary to a region adjacent to a 3' end of a target base sequence of a double-stranded target nucleic acid molecule and a chemically modified 3' end;
(ii) a circular single-stranded DNA comprising a target base sequence;
(iii) a strand displacement-type DNA polymerase; and
(iv) dNTP.

In addition, the present invention provides
a single-stranded target nucleic acid molecule detection kit, comprising:
(i) a primer having a base sequence complementary to a region adjacent to a 3' end of a target base sequence of a single-stranded target nucleic acid molecule and a chemically modified 3' end;
(ii) a primer having a base sequence of a region adjacent to a 3' end of a region different from a region of the target base sequence of the target nucleic acid molecule and having a chemically modified 3' end;
(iii) a circular single-stranded DNA comprising the target base sequence;
(iv) a strand displacement-type DNA polymerase; and
(v) dNTP.

### Advantageous Effects of Invention

The present invention makes it possible to obtain a method for amplifying a nucleic acid, the method being capable of specifically amplifying a nucleic acid in a short period of time, and the use of the method can improve detection sensitivity of a nucleic acid.

### Brief Description of Drawings

Fig. 1 illustrates a principle (first step) of a method for amplifying a nucleic acid of the present invention.
Fig. 2 illustrates the correspondence between linear single-stranded DNAs produced in the first stage and a circular single-stranded DNA.
Fig. 3 illustrates a principle (second stage) of a method for amplifying a nucleic acid of the present invention.
Fig. 4 shows regions corresponding to primers F-in, R-in, F-out, and R-out and regions A and B of target sequences in a case where the λ DNA is used as a template.
Fig. 5 shows results of Experiments 1 to 3.
Fig. 6 shows results of Experiments 4 to 6.
Fig. 7 shows results of Experiments 7 to 9.
Fig. 8 shows results of Experiments 10 to 13.
Fig. 9 shows retests of Comparative Experiments 1 to 4.
Fig. 10 shows results of is Experiments 14 and 15.
Fig. 11 shows results of Experiments 16 and 17.
Fig. 12 shows results of Experiments 18 to 21.
Fig. 13 shows results of Experiments 22 to 25.
Fig. 14 shows a result of electrophoresis conducted on a sample of Experiment 3 in Table 5.

### Description of Embodiments

In a method for amplifying a nucleic acid of the present invention, amplification products which have various chain lengths and include a base sequence to be amplified in a single or multiple repetitions can be obtained by a DNA polymerase elongation reaction, and a further DNA polymerase elongation reaction in which these amplification products serve as a template or an origin of replication proceeds in a chain reaction so that the base sequence to be amplified can be efficiently amplified.

A template DNA including a base sequence to be amplified (also referred to as "a target base sequence" in the present description) of the present invention is a single-stranded or double-stranded linear or circular DNA, and the base sequence to be amplified included in the template DNA is a base sequence in a specific region in the template DNA. Furthermore, the method of the present invention can be carried out by using cDNA, which is obtained by synthesizing a base sequence complementary to RNA molecules using a reverse transcriptase, as a template DNA. In this case, the base sequence to be amplified of the present invention is a base sequence in which uracil (U) in the RNA molecule is substituted by thymine (T).

A primer pair used for the amplification method of the present invention comprises a primer having a base sequence complementary to a region adjacent to a 3' end of a target base sequence to be amplified in the template DNA, and may be anything with which a region sandwiched by the primer pair can be obtained as a linear DNA fragment on the basis of the template DNA. Accordingly, in the case where the template DNA is a double-stranded DNA, primers of the primer pair have target base sequences on respective strands. In the case where the template DNA is a single-stranded DNA, one of the primers of the primer pair has a target base sequence in a region different from a base sequence to be amplified which is present on the template DNA strand.

Here, the primer pair of the present invention comprises a primer having a chemically modified 3' end. When a DNA polymerase elongation reaction is performed by using the primer having the chemically modified 3' end as an origin, a DNA fragment having a chemically modified base residue at an internal position is produced. When a further DNA polymerase elongation reaction is performed by using the DNA fragment as a template, a DNA polymerase cannot add a corresponding base at the position of the chemically modified base. Accordingly, the DNA elongation stops just before the chemically modified base (this is described later with reference to drawings).

The chemical modification used in the present invention is not particularly limited, as long as a DNA polymerase elongation reaction using a DNA fragment subjected to the chemical modification as a template can be stopped as described above. The chemical modification can be selected as appropriate by those skilled in the art. In the present invention, the chemical modification can be performed by changing a base on the primer at the 3' end to an RNA residue, an LNA (Locked Nucleic Acid) residue, or an ENA (2'-O, 4'-C-Ethylene Bridged Nucleic Acid) residue, or to an inosine residue. The number of chemically modified bases at the 3' end is not particularly limited, and, for example, one, two, or three bases from the 3' end can be chemically modified. In general, the present invention can be carried out, as long as only one endmost base at the 3' end is chemically modified.

In the primer pair used in the present invention, it is sufficient that at least one of the two primers constituting the primer pair is chemically modified, and it is preferable that both the two primers are chemically modified.

The method of the present invention can be carried out by using at least one primer pair. In the case of using multiple primer pairs, there is no upper limit to the number; however, for example, a nested design may be adopted. For example, the number is in a range from 1 to 5 pairs, preferably in a range from 1 to 3 pairs, more preferably in a range from 2 to 3 pairs, and especially preferably 2 pairs.

It is preferable that the individual primers do not include a sequence identical or complementary to the other primer in a pair or a primer of other primer pair.

A relative distance (number of bases) between regions to which primers in a pair bind is not particularly limited as long as the reaction of the present invention can proceed. However, it is preferable that the relative distance between the regions to which primers of a primer pair located innermost bind is smaller, and also that the relative distances between the regions to which a primer pair located outside thereof and an adjacent inside primer pair bind are smaller. The relative distance (number of bases) is preferably in a range from 5 b to 1 kb, and more preferably in a range from 10 to 100 b.

A primer to be used in the present invention is not particularly limited as long as the primer can form a complementary double strand with the template DNA; however, the primer is preferably in a range from 8 to 40 mer, and more preferably in a range from 16 to 25 mer. Furthermore, as for properties generally taken into consideration in primer design, such as GC content and secondary structure forming propensity of a template DNA (target base sequence) or the primer, they are also within a range of common technical knowledge of a person skilled in the art. Such a primer design and synthesis can be carried out by using a technique, such as a design program commonly used in the present technical field.

In the case where cDNA obtained by synthesizing a base sequence in an RNA molecule by using a reverse transcriptase is used as a template DNA, a primer pair can also be designed in a similar manner as described above depending on whether the RNA molecule is double stranded or single stranded.

A circular single-stranded DNA used in the method of the present invention comprises the base sequence to be amplified.

The circular single-stranded DNA may be designed to include the base sequence to be amplified (i.e., the target base sequence) in multiple repetitions at constant intervals, or, when both of the primers in the primer pair are chemically modified, their respective base sequences to be amplified may be included in a single or multiple repetitions.

In the circular single-stranded DNA, base sequences in regions other than the target sequence is not particularly limited as long as the reaction of the present invention proceeds specifically; however, it is preferably smaller. Furthermore, in the case of using a strand displacement-type DNA polymerase which has no 3' to 5' exonuclease activity, it is preferable to add one thymine (T) to the 5' end of a base sequence identical to a primer.

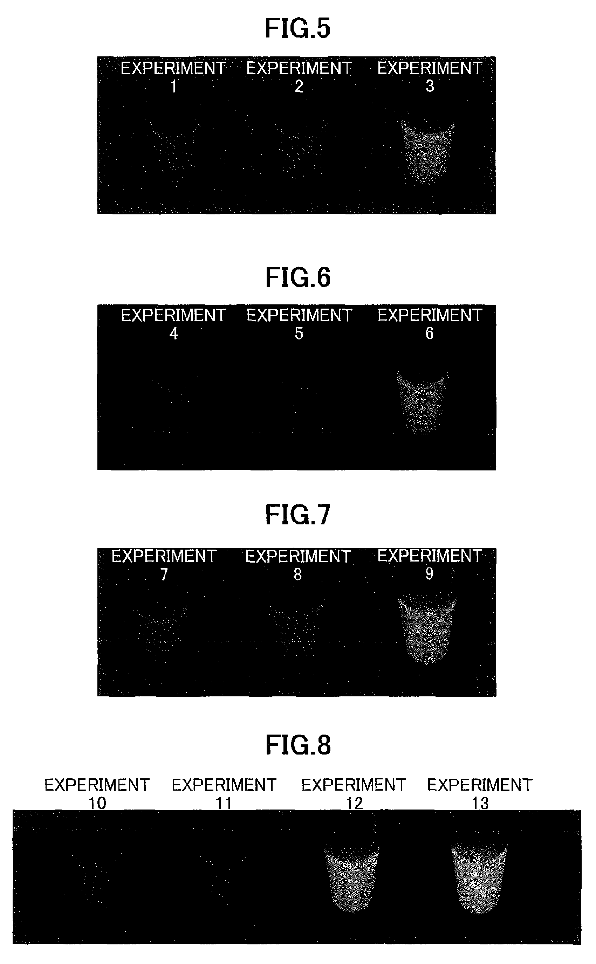

A chain length of the circular single-stranded DNA is not particularly limited as long as the reaction of the present invention proceeds specifically; however, it is preferably in a range from 16 b to 100 kb, and more preferably in a range from 20 to 100 b. Design and synthesis of such a circular single-stranded DNA can be carried out by using a technique, such as a design program commonly used in the present technical field. Furthermore, a circular single-stranded DNA can also be manufactured by a method for manufacturing a circular single-stranded DNA shown in Fig. 6 of International Patent Application Publication No. W02008/026719.

A DNA polymerase used in the method of the present invention has a strand displacement-type 5' to 3' DNA polymerase activity, and is preferably one capable of correctly synthesizing a complementary strand of a base pair. Furthermore, the DNA polymerase may have 3'-5' exonuclease activity, but preferably has no 3'-5' exonuclease activity, and preferably has no 5'-3' exonuclease activity. An optimum temperature of a DNA polymerase is preferably in a range from 50 to 90°C, and more preferably in a range from 60 to 72°C. As such a DNA polymerase, for example, Bst DNA polymerase large fragment derived from Bacillus stearothermophilus, Deep Vent_{R}^{(R)} (exo-) DNA polymerase derived from a bacterium belonging to the genus Pyrococcus, 9 degrees North DNA polymerase derived from a bacterium belonging to the genus Thermococcus (manufactured by New England Biolabs Incorporation), Herculase^{(R)} II Fusion DNA polymerase (manufactured by STRATAGENE), Vent_{R}^{(R)} (exo-) DNApolymerase derived from a strain of the genus Thermococcus, Therminator^{™} DNA polymerase, Therminator^{™}II DNA polymerase (manufactured by New England Biolabs Incorporation), KOD Dash DNA polymerase (manufactured by Toyobo, Co., Ltd), and the like are available by itself or in a kit. Two kinds or more DNA polymerases can be used together as long as they do not interfere with the enzymatic activity of the other.

For an elongation reaction by DNA polymerase, a buffer generally used for nucleic acid amplification (including salts, such as Tris-HC1, KC1., (NH₄)₂SO₄, and MgSO₄), for example, one having an optimized composition provided with a DNA polymerase to be used, may be used.

By adding a template DNA, a primer pair of the present invention, a circular single-stranded DNA of the present invention, a strand displacement-type DNA polymerase, and four kinds of deoxyribonucleoside triphosphates (dNTP: dATP, dCTP, dGTP, and dTTP) which serve as substrates to an appropriate buffer described above, and maintaining this reaction solution at a temperature at which the strand displacement-type DNA polymerase retains its enzymatic activity, for example, at a temperature in a range from 50 to 90°C, preferably in a range from 60 to 72°C, it is possible to efficiently amplify a nucleic acid in a single operation in a single container without applying temperature cycle control. In the meantime, in the case of using multiple primer pairs, it is possible to set a reaction temperature condition of the present invention to a lower condition, for example, in a range from 30 to 50°C. A temperature condition for a DNA polymerase elongation reaction depends on a temperature at which a strand displacement-type DNA polymerase to be used retains its activity. It is not necessarily required to maintain a uniform temperature (constant temperature) throughout the amplification reaction as long as the temperature is within a temperature range in which a strand displacement-type DNA polymerase to be used retains its enzymatic activity; however, it is preferable to maintain a uniform temperature (constant temperature) throughout the amplification reaction (under a constant temperature condition).

Amplification of a nucleic acid can be checked by a precipitation of magnesium pyrophosphate generated in a reaction solution when a nucleic acid is amplified in a DNA polymerase elongation reaction (International Patent Application Publication No. WO2001/083817). Alternatively, it is also possible to check the amplification of a nucleic acid from the presence of fluorescence or the intensity thereof by using a nucleic acid staining fluorescent dye, for example, ethidium bromide, SYBR green (manufactured by Lonza), or the like, which is commonly used in the present technical field, according to a common procedure.

Next, with reference to drawings, an aspect of a method for amplifying a nucleic acid of the present invention will be specifically described.

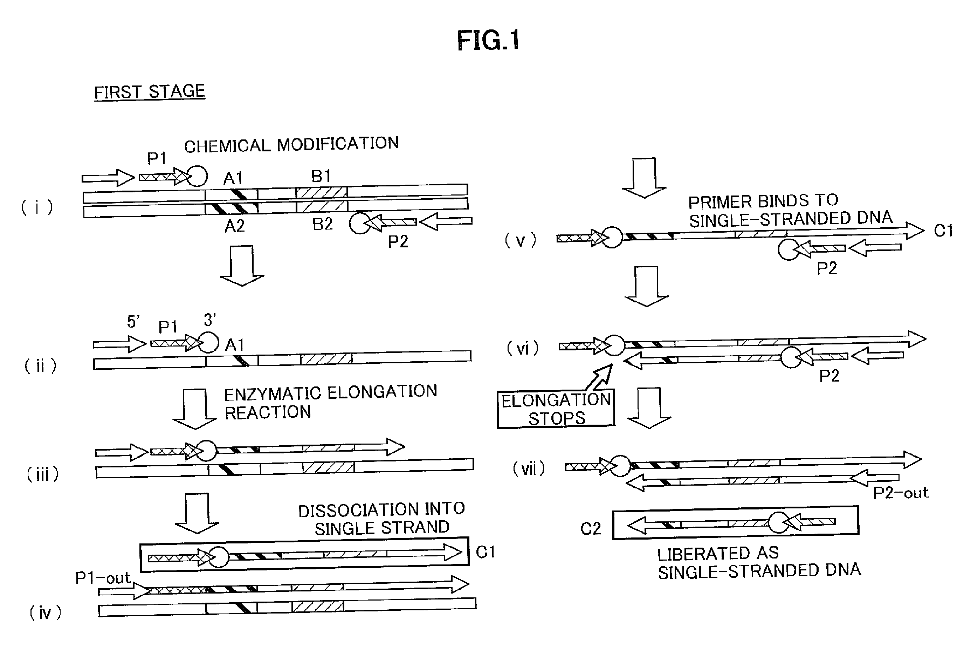

First, a linear DNA fragment can be obtained in a first stage by a DNA polymerase elongation reaction using a template DNA comprising a specific base sequence to be amplified and a primer pair (Fig. 1).

In the case where two regions A and B having a specific base sequence in a double-stranded template DNA are to be amplified, respective sequences of the regions on one strand are defined as A1 and B1, and respective sequences on the other strand are defined as A2 and B2 (Fig. 1, (i)). Among the primer pair, one primer P1 is designed to have a base sequence complementary to a region adjacent to the 3' end of A1, and the other primer P2 is designed to have a base sequence complementary to a region adjacent to the 3' end of B2. In Fig. 1, the modifications of the 3' ends of the primers P1 and P2 are indicated by circles.

A DNA polymerase elongation reaction is performed by using the primer pair and the template DNA. For simplifying the description, attention is focused on only a reaction on the strand (the upper strand in the double-stranded template DNA in Fig. 1) comprising the regions A1 and B1 in the following description. First, the primer P1 binds to the region on the 3 end side of the sequence A1 (Fig. 1, (ii)). After that, by an elongation reaction, a fragment C1 is produced in which a base sequence identical to A2 is synthesized consecutively to a sequence of P1, and then a base sequence identical to B2 is synthesized with an interval (Fig. 1, (iii)). The produced fragment can be dissociated into a double strand by being naturally separated from the template DNA under the elongation reaction conditions due to an action of the enzyme (see, for example, Example 1 in International Patent Application Publication No. WO2008/026719). Alternatively, another primer P1-out is designed and annealed to the outside (the 5' end side) of the primer P1, and elongation is carried out in a similar manner. Thereby, the DNA fragment synthesized from the primer P1 can be peeled from the template DNA, and efficiently dissociated into a single strand (Fig. 1, (iv)).

Next, the primer P2 anneals to the thus amplified fragment C1 serving as a template, and a DNA elongation reaction is performed in the reverse direction (Fig. 1, (v)). Here, the DNA fragment serving as the template includes a chemical modification on the 5' end side of the sequence A2 , Hence, no elongation occurs at the part, and the DNA elongation stops just before the base having a chemical modification (Fig. 1, (vi)). Thus, a DNA fragment C2 is formed. As described for Fig. 1, (iv), the produced DNA fragment C2 can be dissociated into a double strand by being naturally separated from the template DNA under the elongation reaction conditions. Alternatively, the DNA fragment synthesized from the primer P2 can be peeled off from the template DNA and efficiently dissociated into a single strand by adding an outside primer P2-out to the reaction simultaneously as in the case with (iv) (Fig. 1, (vii)),

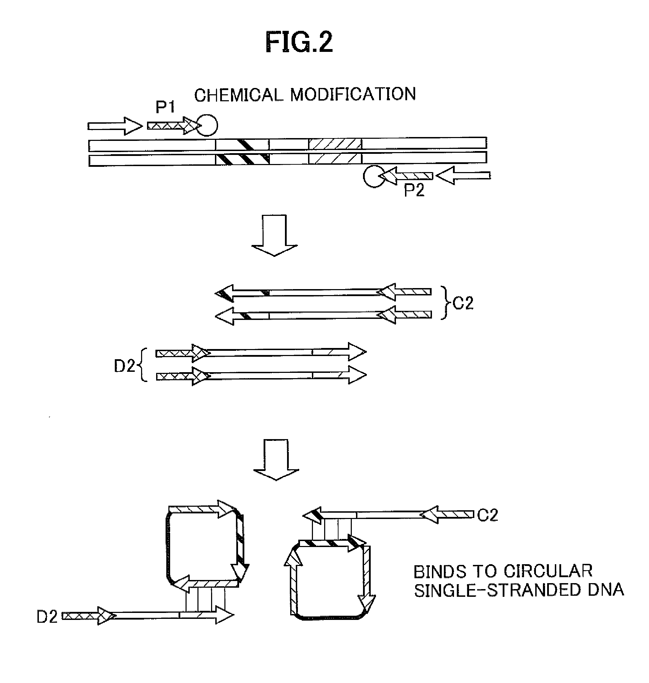

By the above-described reaction, a linear DNA fragment can be obtained which has a sequence of the primer P2 at the 5' end, a sequence consecutive thereto and identical to B,1 and, with an interval, a sequence identical to A1, where the elongation stops. As described above, this shows the case where the upper strand in Fig. 1, (i) is used as a template. Likewise, when the lower strand is used as a template, a linear DNA fragment D2 can be obtained in such a manner that a fragment D1 is first produced by using the primer P2 as a template (not illustrated), and then a DNA elongation using the fragment D1 as a template stops midway. Fig. 2 shows how linear DNA fragments C2 and D2 are produced from the two strands in the double-stranded DNA serving as the template, and how the linear DNA fragments C2 and D2 bind to a circular single-stranded DNA in the second stage to be described later. The obtained linear DNA fragments C2 and D2 serve as origins of replication when an amplification reaction of the second stage starts.

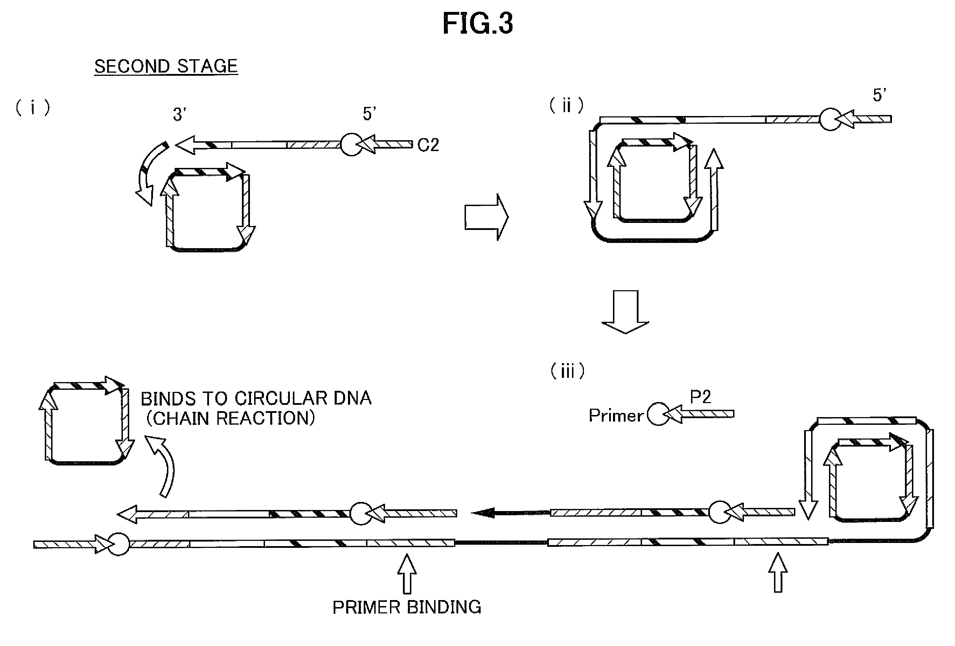

The circular single-stranded DNA used as a template in the second stage is designed to include the target base sequences A2 and/or B1 (Fig. 3, (i)). When such a circular single-stranded DNA is used, both the linear DNA fragments C2 and D2 obtained in the first stage anneals to the circular DNA (see Fig. 2), and a chain amplification reaction can be performed in which the circular single-stranded DNA serves as the origin. Hereinafter, for simplifying the description, description is given of only the case where the linear DNA fragment C2 serves as an origin of replication. A circular single-stranded DNA comprising base sequences identical to A2, B1, and P2, respectively, is shown in Fig. 3. As described above, each linear DNA fragment C2 obtained in the first stage has the base sequence (A1) complementary to the target sequence at the 3' end. Hence, the 3' end region (A1) of the linear DNA fragment anneals to a region of the base sequence (A2) in the 3' end portion of the circular single-stranded DNA, and serves as an origin of replication. Then, a DNA polymerase elongation reaction occurs with the circular single-stranded DNA serving as a template (Fig. 3, (i)). The synthesized DNA strand is elongated in a rolling circle pattern while being peeled off by the strand displacement-type DNA polymerase (Fig. 3, (ii)). The primer P2 in the reaction solution anneals to the DNA strand having been peeled off and elongated and serves as an origin of replication, and then a DNA polymerase elongation reaction occurs. The DNA strand synthesized with the primer as an origin of replication and peeled off anneals to a primer or a circular single-stranded DNA in the reaction solution, and a DNA polymerase elongation reaction occurs (Fig. 3, (iii)).

The process in the first stage and the process in the second stage which are described above proceed simultaneously in parallel once after a linear DNA fragment which triggers the initiation of the second stage is synthesized. Then, a series of amplification reactions proceeds as described above, and, as a result, DNA strands which comprises a specific base sequence to be amplified in a single or multiple repetitions and have various chain lengths are synthesized.

Another aspect of the present invention is a method for detecting a target gene based on the principle of the above-described method for amplifying a nucleic acid. The detection method can be carried out in various scenes in various industries according to an object of detection, an origin of a sample to be subjected to detection, a target gene, and the like.

A sample from which a target gene is to be detected, for example, may be prepared from a body fluid or a tissue fragment derived from human or other animals, may be prepared from soil, ocean water, or a plant derived from the environment, or may be a beverage, a food item, or a medical product manufactured and processed in a factory. These can be used as a sample to be subjected to detection by the detection method of the present invention directly or if needed after being prepared as a nucleic acid (DNA, RNA) extract by an appropriate operation. Such sample preparation can be carried out according to a standard technique which is used in a general nucleic acid detection method.

A target gene may be specific to a microorganism (bacterium, fungus, or the like), an allergen (for example, tick, pollen, or the like), or a virus, or may be a wild-type or a mutant-type gene in a genome of human or other animals.

In the present detection method, "a template DNA" and "a base sequence to be amplified" in the above-described method for amplifying a nucleic acid are replaced by "a target nucleic acid molecule (that is, target gene)" and "a target base sequence in a target gene" of the present detection method, respectively, and the present detection method utilizes the fact that, in the case where a target nucleic acid molecule (that is, a target gene) is present in a sample, a nucleic acid of the target base sequence in the target gene can be amplified on the basis of the same principle as in the above-described method for amplifying a nucleic acid.

In the case where a target nucleic acid molecule is RNA, an enzymatic reaction is performed by further adding a reverse transcriptase (RNA-dependent DNA polymerase) to a sample in the step of the enzymatic reaction in step (a) of the detection method of the present invention, and it is checked whether or not a nucleic acid can be amplified with a cDNA strand, which is synthesized from an RNA molecule in the sample, as a template, as in the case with the above-described method for amplifying a nucleic acid. In the case where a nucleic acid has been amplified, the presence of a target RNA molecule can be detected by determining that the target RNA molecule is present.

Amplification of a nucleic acid can be checked in a similar manner as described above by using the presence of precipitation or fluorescence as an indicator with the use of a precipitation of magnesium pyrophosphate or by using a nucleic acid staining fluorescent dye or the like commonly used in the present technical field. Furthermore, by using an amount of magnesium pyrophosphate generated or intensity of fluorescence as an indicator, an amount of nucleic acid of a target gene present in a sample subjected to detection can also be compared.

As described above, by using the present detection method, even if only a minute amount of nucleic acid of a target gene is present in a sample, it is possible to efficiently amplify and detect a specific base sequence in the target gene in a single operation in a single reaction container. The present detection method may be performed in a reaction container, for example, a micro tube, a microwell plate, a microchip, or the like, and a technique, such as HTS (High-Throughput Screening), may be adopted.

The present invention will be concretely described hereinafter with reference to Examples.

### Examples

The present invention will be concretely described hereinafter with reference to Examples. Chemicals without a company name denoted thereafter are manufactured by Wako Pure Chemical Industries, Ltd. Furthermore, unless otherwise specifically stated, the solvent was water.

### TE buffer

4 mmol/l Tris-HCl (tris-hydroxymethyl-aminomethane) hydrochloric acid
1 mmol/l Ethylene diamino tetraacetic acid, disodium salt, dihydrate (EDTA)

### Adjusted to pH 8.0

1.5% TAE electrophoresis gel
1.5% Agarose
4 mmol/l Tris-HC1
1 mmol/l EDTA
1 mmol/l Acetic acid
LB culture medium
Peptone 1.0%
Yeast Extract 0.5%
Sodium chloride 1.0%
After dissolving, the pH was adjusted to 7.0.

### [Synthesis of a circular single-stranded DNA]

Enzyme products and kit products described below were used in accordance with the respective manuals unless otherwise specifically stated.

Primers used for synthesis of a circular single-stranded DNA are shown in Tables 1 and 2.
[Table 1]

**Table 1: Primer sequences used for synthesis of circular single-stranded DNA**

| Name | Sequence (5' to 3') |
|---|---|
| C60 | AGAGCTATGA TACGCCACTGTCCCT TTTGTAATGTCCGCT GAGAAGGACG CACACATGAT |
| Bind | TCATAGCTCT ATCATGTGTG |

| | |
|---|---|
| ^{*} The 5' end of C60 and the 3' end of Bind were phosphorylated. Both C60 and Bind were purified by HPLC. | |

[Table 2]

**Table 2: Primer sequences used for synthesis of circular single-stranded DNA (Conventional method)**

| Name | Sequence (5' to 3') |
|---|---|
| 60C | AGAGCTATGA CCACCGACCATCTATGACTG GGCATGCACC GAGAAGGACG CACACATGAT |
| Bind | TCATAGCTCT ATCATGTGTG |

| | |
|---|---|
| ^{*}The 5' end of 60C and the 3' end of Bind were phosphorylated. Both 60C and Bind were purified by HPLC. | |

The primer sequences described in Table 1 are primer sequences for synthesis of a circular single-stranded DNA used in Examples of the present invention, and primer sequences used in Examples in Description. Meanwhile, the primer sequences described in Table 2 are primer sequences for synthesis of a circular single-stranded DNA used for carrying out a conventional method, i.e., the invention described in International Patent Application Publication No. WO2008/026719, and primer sequences used in Comparative Examples in DESCRIPTION.

For each of Tables 1 and 2, two primers (1.0 µmol/l each) in the table were mixed with 9°N DNA Ligase (New England biolabs), and a reaction was performed at temperatures shown below:

| | |
|---|---|
| 98°C, 1 minute | -(1) |
| 98°C, 30 seconds | -(2) |
| 90 to 45°C, cooled stepwise over 40 minutes | -(3) |

15 cycles of (2) to (5)

| | |
|---|---|
| 50°C, 10 minutes | -(4) |
| 65°C, 10 minutes | -(5) |
| 4°C, ∞ | -(6) |

After the reaction was completed, Gen Toru Kun (Takara Bio Inc.) was added, and ethanol precipitation was performed. Thereafter, the precipitates were washed twice with 75% ethanol and once with 99.5% ethanol, then dried at 85°C, and dissolved in ultrapure water at 1.0 µmol/l.

After the dissolution, a buffer dedicated to RecJf (New England biolabs) was added, and the mixture was heated at 98°C for 1 minute, and then cooled on ice. Thereafter, RecJf (New England biolabs) was added, and a reaction was performed at 37°C for 2 hours. Then, Gen Toru Kun was added, and ethanol precipitation was performed. Thereafter, the precipitates were washed twice with 75% ethanol, and once with 99.5% ethanol, then dried at 85°C, and dissolved in a TE buffer at 1.0 µmol/l. Thus, a circular single-stranded DNA liquid preparation was obtained. Here, the circular single-stranded DNA liquid preparations obtained by using the primer set in Table 1 and the primer set of Table 2 were termed as a circular single-stranded DNA liquid preparation (1), and a circular single-stranded DNA liquid preparation (2), respectively, which were used for experiments to be described later.

### [Specific gene amplification]

Primers used for gene detection are shown in Table 3. All the primers were purified by HPLC. In addition, bases in the square brackets represent bases subjected to chemical modifications, and represent RNA (manufactured by Greiner), LNA (manufactured by Greiner), or ENA (manufactured by SIGMA) depending on the chemical modification used in Example.
[Table 3]

**Table 3: Primer sequences used for gene detection**

| Name | Sequence (5' to 3') |
|---|---|
| F-in | CCACCGACCATCTATGACT [G] |
| R-in | GGCATGCACCGAGAAGGAC [G] |
| F-out | GTGCGGGTGTTGAATGATTT |
| R-out | GCTAACCAATTCCTAGGCAG |

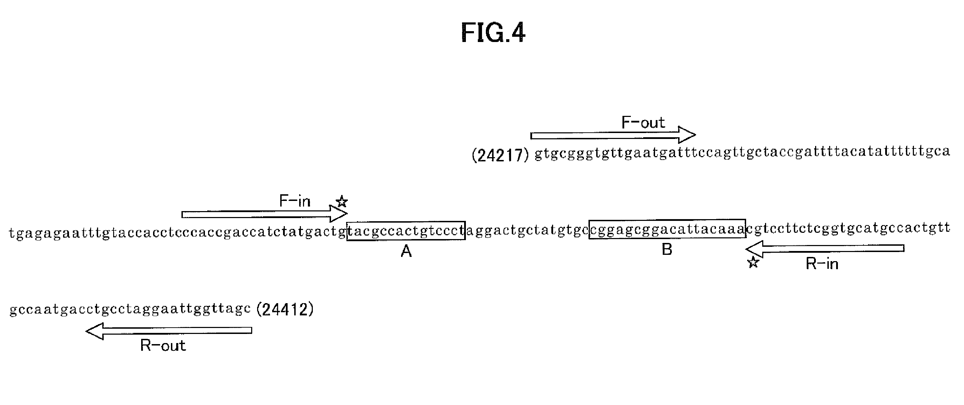

Fig. 4 shows the relationship of the four primers on the λ DNA used as a template. Fig. 4 shows a base sequence from position 24217 to 24412 of the λ DNA (the numbering is done according to Accession No. NC_001416 in the NCBI data base). In addition, regions A and B in Fig. 4 represent regions comprising target base sequences to be amplified. These sequences are present in C60 of the circular single-stranded DNA shown in Table 1 (bases at positions 11 to 25 of C60 correspond to A, and bases at positions 26 to 40 correspond to a sequence complementary to B).

In addition, as for the sequence of 60C used in experiments of a conventional method for comparison, bases at positions 11 to 30 of 60C are identical to those in the sequence of primer F-in, and bases at positions 31 to 50 are identical to those in a sequence complementary to the primer R-in. Here, since linear DNA fragments of the conventional method are produced for regions including the entireties of F-in and R-in, the circular DNA was designed to include sequences corresponding to the 3' ends (i.e., origins of replication) of the fragments.

Meanwhile, when inosine base was employed as the chemical modification, the primers described in Table 4 were used, where the following primers FI-in and RI-in were used instead of F-in and R-in in Table 3 (F-out and R-out were the same).
[Table 4]

**Table 4: Primer sequences used for gene detection**

| Name | Sequence (5' to 3') |
|---|---|
| FI-in | CCACCGACCATCTATGACT I |
| RI-in | GGCATGCACCGAGAAGGAC I |
| F-out | GTGCGGGTGTTGAATGATTT |
| R-out | GCTAACCAATTCCTAGGCAG |

| | |
|---|---|
| ^{*}I represents an inosine base (manufactured by Greiner). | |

For a gene detection reaction, the following were used. Enzyme: 4 U/25 µl Bst DNA polymerase (New England Biolabs Incorporation)
Template: λ genome (Takara Bio Inc.) 1.0 µl/20 µl
Bacillus subtilis was obtained by culturing Bacillus subtilis in an LB culture medium, and then by using a genome extraction instrument (12 PLUS manufactured by Precision System Science Co., Ltd.).
Reaction buffer

| | |
|---|---|
| 1/10 volume | a buffer provided with Bst DNA polymerase |
| 0.4 M | betaine |
| 1.5 µmol/l | primers (F-in and R-in) in Table 3 |
| 0.5 µmol/l | primers (F-out and R-out) in Table 3 |
| 600 µmol/l each | dNTP |
| 0.1 µg/ml | circular single-stranded DNA liquid preparation (1) or (2) |

The reagents were mixed under the conditions in Table 3, heated at 98 °C for 1 minute, cooled on ice, added with the enzyme, and then incubated at 63°C for 60 minutes.

### [Check of DNA amplification]

A nucleic acid staining reagent SYBR GreenI (Takara Bio Inc.) was diluted to 1/10, 1 µl thereof was added to 20 µl of the sample, and observation was performed at a wavelength of 302 nm with a highly-sensitive filter.

### A. Cases where RNA was used for chemical modification

The results of DNA amplification are shown in the following Table 5, and Figs. 5 and 6.
[Table 5]

**Table 5**

| | Enzyme | λ Genome (Template) | Bacillus subtilis | Reaction Buffer | Nucleic acid Amplification |
|---|---|---|---|---|---|
| Experiment 1 | × | O | × | ○ | × |
| Experiment 2 | ○ | × | × | ○ | × |
| Experiment 3 | ○ | ○ | × | ○ | ○ |
| Experiment 4 | × | ○ | ○ 10⁶ | ○ | × |
| Experiment 5 | ○ | × | ○ 10⁶ | ○ | × |
| Experiment 6 | ○ | ○ | ○ 10⁶ | ○ | ○ |

According to the above results, rapid nucleic acid amplification was observed in the samples comprising the enzyme and the template (Experiments 3 and 6), whereas no nucleic acid amplification was observed in the samples comprising the enzyme but no template (Experiments 2 and 5). Furthermore, no nucleic acid amplification was observed even in the sample comprising DNA other than the target sequence (Experiment 5). Hence, it was found that the sequence was specifically recognized. Note that no nucleic acid amplification was performed for the samples comprising no enzyme (Experiments 1 and 4).

The present invention makes it possible to specifically recognize a nucleic acid sequence and to perform rapid nucleic acid amplification.

### B. Cases where LNA was used for chemical modification

Results of DNA amplification are shown in Table 6 and Fig. 7.
[Table 6]

**Table 6**

| | Enzyme | λ Genome (Template) | Reaction Buffer | Nucleic acid Amplification |
|---|---|---|---|---|
| Experiment 7 | × | ○ 10⁶, | ○ | × |
| Experiment 8 | ○ | × | ○ | × |
| Experiment 9 | ○ | ○ 10⁶ | ○ | ○ |

According to the results, it was found that similar reactions occurred in Experiments 1 to 3 in Table 5 and Experiments 7 to 9 in Table 6. The present invention provides an excellent effect not only when RNA is used, but also LNA is used.

### C. Comparison between cases were chemical modification was performed with LNA and the conventional method where no chemical modification was performed.

In experiments 10 to 14, DNA was amplified by using the primers F-in and R-in having the 3' ends modified with LNA and the circular single-stranded DNA (circular single-stranded DNA liquid preparation (1)) obtained by circularization of C60 in Table 1 as a template, as in the case with the experiments of B. Meanwhile, in Comparative Experiments 1 to 4, DNA was amplified by using primers having 3' ends which were not chemically modified, and the circular single-stranded DNA (circular single-stranded DNA liquid preparation (2)) obtained by circularization of 60C in Table 2 as a template.

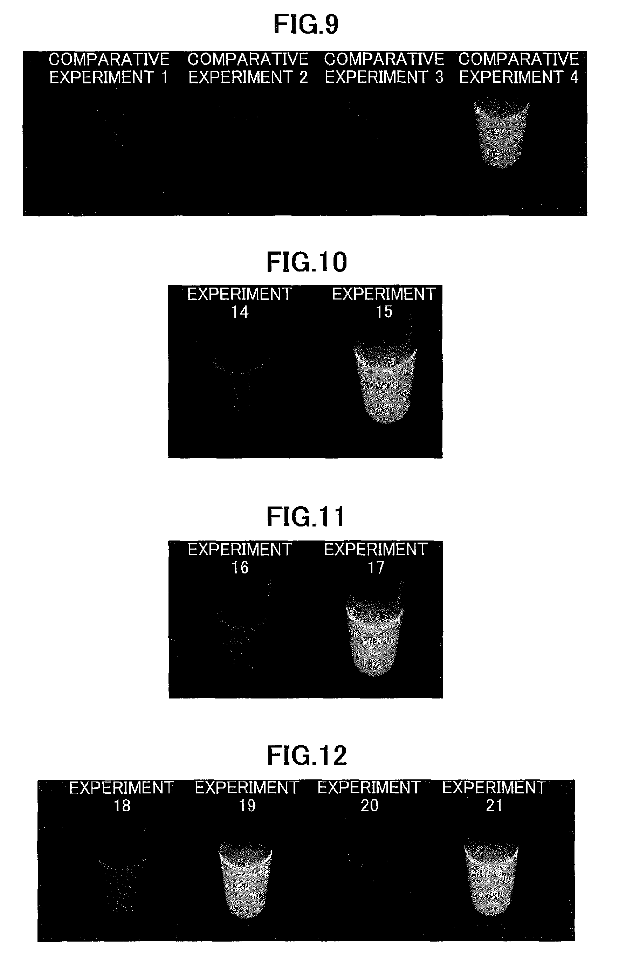

The results of the DNA amplification are shown in Table 7 and Figs. 8 and 9.
[Table 7]

**Table 7**

| | Enzyme | × Genome (Template) | Reaction Buffer | Nucleic acid Amplification |
|---|---|---|---|---|
| Experiment 10 | ○ | × | ○ | × |
| Experiment 11 | ○ | ○ 10⁴ | ○ | × |
| Experiment 12 | ○ | ○ 10⁵ | ○ | ○ |
| Experiment 13 | ○ | ○ 10⁶ | ○ | ○ |
| Comparative Experiment 1 | ○ | × | ○ | × |
| Comparative Experiment 2 | ○ | ○ 10⁴ | ○ | × |
| Comparative Experiment 3 | ○ | ○ 10⁵ | ○ | × |
| Comparative Experiment 4 | ○ | ○ 10⁶ | ○ | ○ |

According to the above results, the detection sensitivity of the conventional method was 10⁶ (Comparative Experiment 4), whereas the detection sensitivity was improved to 10⁵ (Experiments 12 and 13). Both the methods did not detect 10⁴ (Experiment 11, and Comparative Experiment 2). In addition, no nucleic acid amplification was performed for the samples comprising no template (Experiment 10 and Comparative Experiment 1).

According to the above results, it was shown that the use of the present invention increased the detection sensitivity by approximately 10 times or more as compared with the conventional method.

### D. Cases where ENA or inosine base was used for chemical modification

A reaction buffer used for cases where inosine base was used for chemical modification is as follows.

### Reaction buffer

- 1/10 volume: a buffer provided with Bst DNA polymerase
- 0.4 M: betaine
- 1.5 µmol/l: primers in Table 3
(In the cases of ENA: F-in and R-in, in the cases of inosine residue: FI-in and RI-in)
- 0.5 µmol/l: primers in Table 3 (F-out and R-out)
- 600 µmol/l: each dNTP
- 0.1 µg/ml: circular single-stranded DNA liquid preparation (1)

The chemical modification was performed with ENA in Experiments 14 and 15, and with inosine base in Experiments 16 and 17.

The results of the DNA amplification are shown in Table 8 and Figs. 10 and 11.
[Table 8]

**Table 8**

| | Enzyme | λ Genome (Template) | Reaction Buffer | Nucleic acid Amplification |
|---|---|---|---|---|
| Experiment 14 | ○ | × | ○ | × |
| Experiment 15 | ○ | ○ 10⁶ | ○ | ○ |
| Experiment 16 | ○ | × | ○ | × |
| Experiment 17 | ○ | ○ 10⁶ | ○ | ○ |

According to the above results, it was found that efficient DNA amplification reactions occurred also in the cases where ENA was used (Experiments 14 and 15) and the cases where inosine base was used (Experiments 16 and 17), as in the case with Tables 5 and 6.

### E. Cases where only a single sequence capable of binding to a circular single-stranded DNA was present

A circular single-stranded DNA was prepared which comprised only one sequence capable of binding to the linear DNA fragment, and the degree of DNA amplification was checked. Circularization of each of the primers 60F and 60R was performed by using the primer Bind. Note that the 5' end of each of 60F and 60R was phosphorylated and the 3' end of Bind was phosphorylated. All the 60F, 60R, and Bind were purified by HPLC.
[Table 9]

| Table 9: Primer sequences used for synthesis of circular single-stranded DNAs | |
|---|---|
| Name | Sequence (5' to 3') |
| 60F | AGAGCTATGA TACGCCACTGTCCCT TACGCCACTGTCCCT GAGAAGGACG CACACATGAT |
| 60R | AGAGCTATGA TTTGTAATGTCCGCT TTTGTAATGTCCGCT GAGAAGGACG CACACATGAT |
| Bind | TCATAGCTCT ATCATGTGTG |

Bases at positions 11 to 25 of 60F correspond to the sequence in the region A of Fig. 4, and bases at positions 26 to 40 of 60R correspond to a sequence complementary to the region B. Circular DNAs were synthesized in the same manner as in the above-described method, and the occurrence of the reaction was checked by employing the above-described reaction conditions.

In Experiments 22 and 23, DNA amplification was performed by using the circular DNA obtained from 60F as a template. In Experiments 24 and 25, DNA amplification was performed by using the circular DNA obtained from 60R as a template.

The results of the DNA amplification are shown in Table 10 and Fig. 12.
[Table 10]

**Table 10**

| | Enzyme | λ Genome (Template) | Reaction Buffer | Nucleic acid Amplification |
|---|---|---|---|---|
| Experiment 18 | ○ | × | ○ | × |
| Experiment 19 | ○ | ○ 10⁶ | ○ | ○ |
| Experiment 20 | ○ | × | ○ | × |
| Experiment 21 | ○ | ○ 10⁶ | ○ | ○ |

According to the above results, it was found that efficient DNA amplification occurred also when 60F in Table 9 was used (Experiments 18 and 19) and when 60R in Table 9 was used (Experiments 20 and 21), as in the case with Tables 5 and 6.

It has been shown that the method of the present invention makes it possible to perform an efficient DNA amplification, as long as at least one site capable of binding to the circular DNA is present.

### F. Highly-sensitive conditions using 9 degrees North DNA polymerase and LNA

**[Table 11]**

| Primer sequences used for synthesis of circular single-stranded DNA | |
|---|---|
| Name | Sequence (5' to 3') |
| C65 | AGAGCTATGA TACGCCACTG TCCCTTTTGT AATGTCCGCT GACTGGAGAA GGACGCACAC ATGAT |
| Bind-2 | TCATAGCTCT ATCATGTGTG [G] |

| | |
|---|---|
| ^{*}The 5' end of C65 and the 3' end of Bind-2 were phosphorylated. Both C65 and Bind-2 were purified by HPLC. In addition, the 3' end of Bind-2 was modified with LNA. | |

### A circular single-stranded DNA was synthesized by the same method as described above.

**[Table 12]**

| Primer sequences used for gene detection | |
|---|---|
| Name | Sequence (5' to 3') |
| F-in | CCACCGACCATCTATGACT [G] |
| R-in | GGCATGCACCGAGAAGGAC [G] |
| F-out | GTGCGGGTGTTGAATGATT [T] |
| R-out | GCTAACCAATTCCTAGGCA [G] |

| | |
|---|---|
| The 3' ends of all the primers used for the reaction were modified with LNA. | |

### Reaction buffer

| | |
|---|---|
| 1/10 volume | A buffer provided with 9 degrees North DNA polymerase |
| 0.4 M | betaine |
| 1.6 µmol/l | primer (F-in) in Table 12 |
| 1.0 µmol/l | primer (R-in) in Table 12 |
| 0.5 µmol/l | primers (F-out and R-out) in Table 12 |
| 600 µmol/l | each dNTP |
| 0.1 µg/ml | circular single-stranded DNA liquidpreparation (1) |
| 0.2 U | 9 degrees North DNA polymerase |

The reagents were mixed under the conditions in Table 3, and a reaction was performed under the following conditions.

| | |
|---|---|
| 98°C, 1 minute | -(1) |
| 98°C, 30 seconds | -(2) |
| 65°C, 1 minute | -(3) |

10 cycles of (2) to (3)

| | |
|---|---|
| 98°C, 30 seconds | -(4) |
| 65°C, 40 minutes | -(5) |
| 4°C, ∞ | -(6) |

The results of the experiments are shown in Experiments 22 to 25.

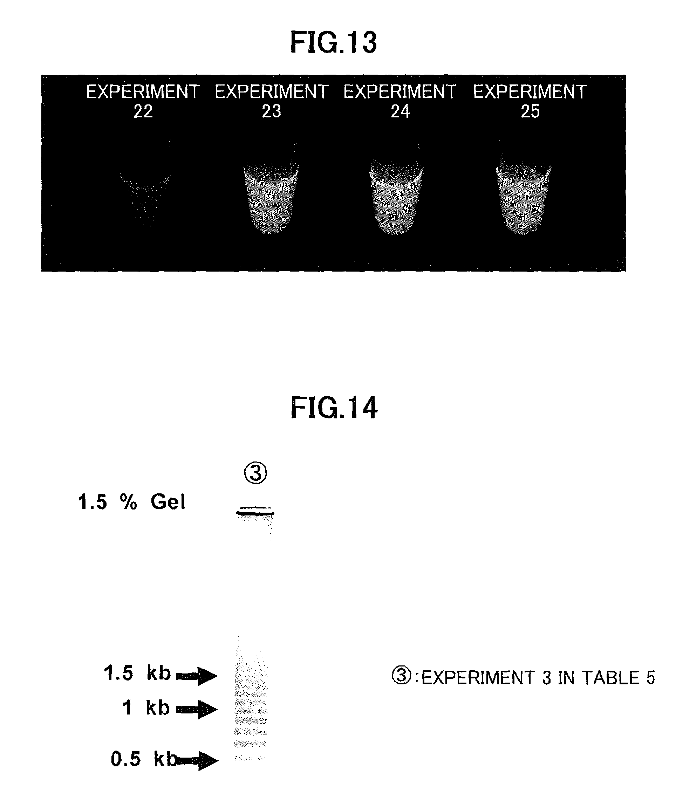

The results of the DNA amplification are shown in Table 13 and Fig. 13.

**[Table 13]**

| | Enzyme | λ Genome (Template) | Reaction Buffer | Nucleic acid Amplification |
|---|---|---|---|---|
| Experiment 22 | ○ | × | ○ | × |
| Experiment 23 | ○ | ○ 10² | ○ | ○ |
| Experiment 24 | ○ | ○ 10³ | ○ | ○ |
| Experiment 25 | ○ | ○ 10⁴ | ○ | ○ |

According to the above results, when reactions were performed by using the circular DNA and the primers in Tables 11 and 12, and 9 degrees North DNA polymerase, with the temperature changed with a thermal cycler, nucleic acid amplification was observed at 10² or more as shown by the result of Experiment 23. Hence, it was found that the sensitivity was increased to 10².

It was found that the sensitivity was improved by changing the temperature cycle according to the method of the present invention.

### G. Check of DNA amplification by electrophoresis

Electrophoresis (100 V, 50 minutes) was performed on the sample of Experiment 3 in Table 5 by using a 1.5% TAE electrophoresis gel and an Electrophoresis bath Mupid-ex (Advance Co., Ltd.).

After that, staining was performed with a nucleic acid staining reagent SYBR GreenI, and a photograph was taken (Fig. 14).

## Claims

1. A method for amplifying a nucleic acid, comprising:
(a) obtaining a linear DNA fragment by performing a DNA polymerase elongation reaction by using a template DNA comprising a base sequence to be amplified and a primer pair comprising a primer having a base sequence complementary to a region adjacent to a 3' end of the base sequence to be amplified and a chemically modified 3' end; and
(b) performing a strand displacement-type DNA polymerase elongation reaction on a circular single-stranded DNA comprising the base sequence to be amplified and serving as a template, with a 3' end of the linear DNA fragment obtained in (a) serving as an origin of replication.

2. The method according to claim 1, wherein the chemical modification is change to an RNA residue, an LNA residue, or an ENA residue or change to an inosine residue.

3. The method according to claim 1 or 2, wherein two primers constituting the primer pair are both chemically modified.

4. The method according to any one of claims 1 to 3, wherein step (a) is performed by further adding a second primer pair composed of two primers, said two primers designed for 5' end side of each primer constituting the primer pair used in step (a).

5. The method according to any one of claims 1 to 4, wherein (a) and (b) are performed under the same temperature condition.

6. The method according to any one of claims 1 to 5, wherein the template DNA comprising the base sequence to be amplified comprises a DNA strand obtained by reverse transcription using RNA as a template.

7. A nucleic acid amplification kit comprising:
(i) a primer pair comprising a primer having a base sequence complementary to a region adjacent to a 3' end of a base sequence to be amplified and a chemically modified 3' end;
(ii) a circular single-stranded DNA comprising the base sequence to be amplified;
(iii) a strand displacement-type DNA polymerase; and
(iv) dNTP.

8. A method for detecting a double-stranded target nucleic acid molecule comprising:
(a) adding a primer pair comprising a primer having a base sequence complementary to a region adjacent to a 3' end of a target base sequence of a target nucleic acid molecule and a chemically modified 3' end; a circular single-stranded DNA comprising the target base sequence; a strand displacement-type DNA polymerase; and dNTP to a sample to be subjected to detection, and performing an enzymatic reaction at a temperature at which the strand displacement-type DNA polymerase retains its activity;
(b) checking whether or not a nucleic acid is amplified in the sample subjected to the enzymatic reaction; and
(c) in the case where a nucleic acid is amplified, determining that a double-stranded target nucleic acid molecule is present in the sample subjected to detection.

9. A method for detecting a single-stranded target nucleic acid molecule comprising:
(a) adding a primer having a base sequence complementary to a region adjacent to a 3' end of a target base sequence of a target nucleic acid molecule and a chemically modified 3' end; a primer having a base sequence of a region adjacent to a 3' end of a target base sequence of a region different from a region of the target base sequence of the target nucleic acid molecule and a chemically modified 3' end; a circular single-stranded DNA comprising the target base sequences; a strand displacement-type DNA polymerase; and dNTP to a sample to be subjected to detection, and performing an enzymatic reaction at a temperature at which the strand displacement-type DNA polymerase retains its activity;
(b) checking whether or not a nucleic acid is amplified in the sample subjected to the enzymatic reaction; and
(d) in the case where a nucleic acid is amplified, determining that a single-stranded target nucleic acid molecule is present in the sample subjected to detection.

10. A double-stranded target nucleic acid molecule detection kit, comprising:
(i) a primer pair comprising a primer having a base sequence complementary to a region adjacent to a 3' end of a target base sequence of a double-stranded target nucleic acid molecule and a chemically modified 3' end;
(ii) a circular single-stranded DNA comprising a target base sequence;
(iii) a strand displacement-type DNA polymerase; and
(iv) dNTP.

11. A single-stranded target nucleic acid molecule detection kit, comprising:
(i) a primer having a base sequence complementary to a region adjacent to a 3' end of a target base sequence of a single-stranded target nucleic acid molecule and a chemically modified 3' end;
(ii) a primer having a base sequence of a region adjacent to a 3' end of a region different from a region of the target base sequence of the target nucleic acid molecule and having a chemically modified 3' end;
(iii) a circular single-stranded DNA comprising the target base sequence;
(iv) a strand displacement-type DNA polymerase; and
(v) DNTP.

12. The detection kit according to claim 10 or 11, further comprising (vi) a reverse transcriptase.
